Europäisches Patentamt

European Patent Office

Office européen des brevets

(19)

(11) EP 0 624 244 B1

(12) FASCICULE DE BREVET EUROPEEN

(45) Date de publication et mention
de la délivrance du brevet:
**05.03.1997 Bulletin 1997/10**

(21) Numéro de dépôt: 93904123.2

(22) Date de dépôt: 27.01.1993

(51) Int Cl.6: **G01N 11/08**, B29C 45/76

(86) Numéro de dépôt international:
**PCT/FR93/00079**

**WO 93/15387 (05.08.1993 Gazette 1993/19)**

(54) **RHEOMETRE POUR PRESSE A INJECTER**

RHEOMETER FÜR SPRITZGUSSPRESSE

RHEOMETER FOR INJECTION PRESS

(84) Etats contractants désignés:
**CH DE FR GB IT LI**

(30) Priorité: **28.01.1992 FR 9200896**

(43) Date de publication de la demande:
**17.11.1994 Bulletin 1994/46**

(73) Titulaire: **CONSERVATOIRE NATIONAL DES
ARTS ET METIERS
F-75003 Paris (FR)**

(72) Inventeurs:
• **HADDOUT, Abdellah
F-93300 Aubervilliers (FR)**
• **VILLOUTREIX, Gilbert
F-78230 Le Pecq (FR)**

(74) Mandataire: **Martin, Jean-Jacques
Cabinet REGIMBEAU
26, Avenue Kléber
75116 Paris (FR)**

(56) Documents cités:
DE-A- 4 001 341        FR-A- 2 621 525
GB-A- 2 131 953        GB-A- 2 158 252

• SIEMENS FORSCHUNGS- UND
ENTWICKLUNGSBERICHTE. vol. 16, no. 3, 1987,
BERLIN DE pages 84 - 89 W. PFANDL 'Rheology
of polymer blends'

# Description

La présente invention concerne un rhéomètre destiné à être disposé en sortie d'une vis d'alimentation d'une presse à injecter un matériau polymère, comprenant un corps de rhéomètre dont une première extrémité est munie d'une pièce de raccordement mécanique à ladite vis d'alimentation, ledit corps de rhéomètre présentant un conduit pour l'écoulement dudit matériau polymère et au moins deux capteurs de pression placés le long dudit conduit.

L'invention trouve une application avantageuse dans le domaine de l'industrie des matières plastiques, et plus particulièrement dans l'injection, l'extrusion et le moulage desdites matières plastiques.

D'une manière très générale, les presses à injecter des matériaux polymères comprennent une trémie qui achemine sous forme de granulés lesdits matériaux polymères vers une vis sans fin d'alimentation. Un dispositif de chauffage porte à l'état fondu les matériaux à injecter qui sont alors conduits par ladite vis d'alimentation vers une buse d'injection sous une vitesse et une pression hydraulique dont les valeurs sont imposées par la presse.

De façon à pouvoir bien maîtriser les conditions de l'injection, il y a avantage à connaître les principaux paramètres rhéologiques d'écoulement du matériau polymère considéré.

Dans ce but, il est déjà connu d'utiliser en sortie de la vis d'alimentation, et à la place de la buse d'injection, un rhéomètre dans lequel deux capteurs de pression placés en amont et en aval de l'écoulement permettent d'effectuer des mesures conduisant à une détermination de la viscosité du matériau polymère fondu à la température de mise en oeuvre. Le rhéomètre est ensuite démonté et la buse d'injection remise en place afin de procéder à l'opération d'injection.

L'utilisation de tels rhéomètres exige de procéder à chaque fois à la dépose et la pose de la buse d'alimentation, d'une part, et du rhéomètre lui-même, d'autre part.

En outre, avec ces rhéomètres, le matériau polymère fondu s'évacue librement vers l'extérieur sous une pression sensiblement égale à la pression atmosphérique, donc notablement plus faible que la pression réelle d'injection en fonctionnement normal. Il s'ensuit là encore une erreur systématique liée au fait que les conditions de mesure sont différentes des conditions effectives d'utilisation.

Il a déjà été proposé dans la demande de brevet FR-2 621 525 un dispositif d'injection de matériaux thermoplastiques sur lequel est monté entre la presse et la buse un module rhéométrique à filière capillaire. Le corps du module est percé par des alésages qui reçoivent des capteurs de pression. Ces alésages sont disposés en amont et en aval de la filière.

Ainsi, les capteurs de pression n'y sont pas placés directement au niveau de l'écoulement dans la filière, mais dans des volumes à l'extérieur du conduit d'écoulement. Les pressions mesurées sont celles de ces volumes et non celles du matériau polymère au niveau de l'écoulement.

Pour éviter ce problème, le document GB-A-2621525 divulgue un dispositif permettant de procéder à des mesures de pression dans la filière.

L'invention propose quant à elle un rhéomètre destiné à être disposé en sortie d'une vis d'alimentation d'une machine de transformation de matériau polymère (telle qu'une presse à injecter ou une extrudeuse), comprenant un corps de rhéomètre dont une première extrémité est munie d'une pièce de raccordement mécanique à ladite vis d'alimentation et dont une deuxième extrémité, opposée à la première, est munie d'une buse d'injection du matériau polymère, ledit corps de rhéomètre présentant un conduit pour l'écoulement dudit matériau polymère et au moins deux capteurs de pression placés le long dudit conduit, ledit conduit étant au moins partiellement constitué par une filière présentant un trou longitudinal cylindrique d'écoulement, caractérisé par les caractéristiques contenues dans la partie caractérisante de la revendication 1.

Une telle structure de rhéomètre présente, par rapport aux rhéomètres de l'art antérieur, de nombreux avantages.

En particulier, elle permet la mesure directe des pressions d'écoulement dans les conditions réelles d'injection. Les paramètres rhéologiques sont ainsi déterminés avec une grande précision, sans qu'il ne soit nécessaire d'effectuer sur les mesures de pression des corrections pour tenir compte de la géométrie des volumes dans lesquels se trouvent les capteurs de pression ou des perturbations dues aux effets d'entrée ou de sortie de la filière (incidence de l'angle d'entrée, compressibilité de la matière, effets élastiques).

En outre, elle autorise le relevé de mesures de pression en trois points ou plus de l'écoulement dans la filière, de sorte qu'il est possible d'avoir accès non seulement à la viscosité du matériau polymère, mais aussi à d'autres paramètres caractéristiques de ce matériau, tels que son élasticité, que les seules mesures de pression en amont et en aval de la filière ne permettaient pas de déterminer.

Les rhéomètres de l'art antérieur ne permettent, quant à eux, que de donner l'ordre de grandeur d'une propriété particulière (contrainte de cisaillement) et ce d'une manière indirecte en se basant sur des hypothèses simplificatrices.

Egalement, cette structure permet d'utiliser des conduits d'écoulement dont le diamètre intérieur est beaucoup plus petit que dans le cas des rhéomètres de l'art antérieur pour lesquels le diamètre de la filière doit être supérieur à un diamètre limite fonction des dimensions des volumes en amont et en aval de la filière. Les taux de cisaillement que permet de réaliser le rhéomètre selon l'invention sont donc plus élevés que dans le cas des rhéomètres de l'art antérieur.

L'invention est avantageusement complétée par les différentes caractéristiques suivantes prises seules ou selon toutes leurs combinaisons techniquement possibles :

- la filière présente au moins trois trous transversaux débouchant dans le trou d'écoulement et recevant des capteurs de pression au débouché dudit trou d'écoulement,

- les capteurs de pression sont reliés à une unité de traitement qui détermine en fonction des mesures de pression fournies par ces capteurs la viscosité apparente et l'élasticité du matériau polymère,

- le diamètre du trou d'écoulement de la filière est inférieur ou égal à 2,5 mm,

- le diamètre du trou d'écoulement de la filière est compris entre 2 et 2,5 mm,

- le corps de rhéomètre comporte en outre au moins un trou de logement destiné à recevoir un capteur de température,

- la pièce de raccordement comporte un trou de logement destiné à recevoir un capteur de température,

- le corps du rhéomètre comporte au moins deux trous de logement destiné à recevoir un capteur de température et débouchant dans le conduit respectivement en amont et en aval de la filière,

La description qui va suivre en regard des dessins annexés, donnés à titre d'exemples non limitatifs, fera bien comprendre en quoi consiste l'invention et comment elle peut être réalisée.

La figure 1 est une vue en coupe d'un rhéomètre conforme à l'invention.

La figure 2 est un schéma d'une unité de traitement des mesures fournies par le rhéomètre de la figure 1.

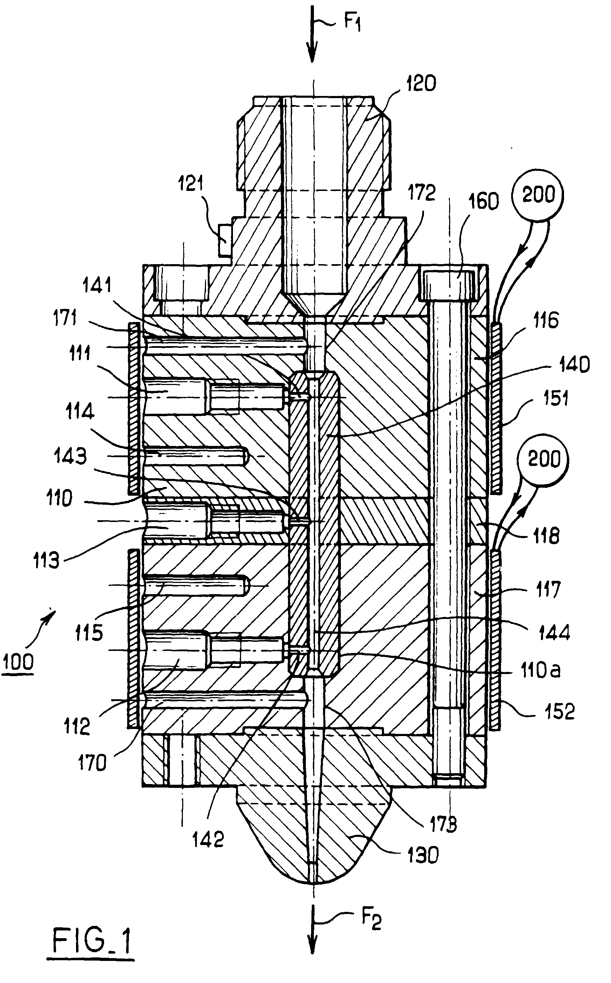

La figure 1 montre, en coupe, un rhéomètre 100, destiné à être disposé en sortie d'une vis d'alimentation, non représentée, d'une presse à injecter un matériau polymère dans la direction représentée par les flèches FI et F2. Le rheomètre 100 de la figure 1 comprend un corps 110 de rhéomètre dont une première extrémité est munie d'une pièce 120 de raccordement mécanique a ladite vis d'alimentation, tandis qu'une deuxième extrémité, opposée à la première, reçoit une buse 130 d'injection dans un moule, non représenté, du matériau polymère à l'état fondu.

Comme on peut le voir sur la figure 1, le corps 110 de rhéomètre comprend un logement cylindrique 110a dans lequel est disposée une filière cylindrique 140 présentant un trou longitudinal 144 d'écoulement qui définit avec des canaux 172, 173 qui le prolongent en amont et en aval de la filière un conduit permettant au matériau polymère de s'écouler depuis la pièce 120 de raccordement à la vis d'alimentation jusqu'à la buse 130 d'injection.

La filière 140 de la figure 1 est munie de trois trous transversaux 141, 142, 143 débouchant dans ledit trou 144 d'écoulement, et dans lesquels peuvent être logées respectivement les extrémités de trois capteurs 111, 112, 113 de pression au débouché du trou d'écoulement. Les capteurs 111, 112, 113 de pression sont, de préférence, des transducteurs piézoélectriques dont l'élément sensible est directement en contact d'affleurement avec le matériau polymère fondu dans le trou d'écoulement de la filière 140. Les extrémités des capteurs 111, 112 et 113 affleurent dans le trou d'écoulement 144 respectivement vers l'entrée et la sortie de la filière, et en sa partie médiane.

La distance séparant l'axe d'un tel capteur 111, 112, 113 de l'entrée ou de la sortie de la filière est supérieure à dix fois le diamètre du trou longitudinal 144. Ces distances minimales sont à respecter pour obtenir une mesure non perturbée des pressions d'écoulement dans la filière. Le diamètre du trou 144 est avantageusement choisi entre 2 et 2.5 mm. Pour une filière de diamètre plus faible, la présence des capteurs entraîne des perturbations de l'écoulement du matériau polymère. Il est à noter que le matériau polymère ne pourrait être passé dans des filières de diamètre compris entre 2 et 2,5 mm si le rhéomètre n'était pas monté sur une presse d'injection.

De façon à maintenir le matériau polymère à l'état fondu à une température de consigne donnée, le corps 110 de rhéomètre est pourvu de moyens de régulation de la température constitués, dans l'exemple de la figure 1, par deux manchons chauffants 151, 152 à résistances dont le courant de chauffage est régulé par un organe 200 de commande situe dans la presse à injecter.

Afin de contrôler la température du rhéomètre, différents points de mesure sont prévus sur le corps 110 de rhéomètre où des trous 114, 115, 170, 171 de logement sont aménagés pour recevoir des capteurs de température, thermocouples par exemple. Les logements 114, 115 sont des trous borgnes débouchant à l'extérieur du corps 110 et ménagés au droit de la filière 140, perpendiculairement à l'axe selon lequel elle s'étend. Ces logements 114 et 115 sont disposés chacun entre le logement d'un capteur 111, 112 d'extrémité et le logement du capteur médian 113. Les logements 170 et 171 sont perpendiculaires à l'axe du conduit du rhéomètre et débouchent, d'une part, à l'extérieur du corps 110 et, d'autre part respectivement, dans les canaux 172, 173 en amont et en aval de la filière. La pièce 120 de raccordement comporte un trou 121 de logement destiné à un capteur de température, également du type thermocouple.

Comme on peut l'observer sur la figure 1, le corps 110 de rhéomètre peut être constitué de trois pièces superposées dans la longueur selon laquelle le conduit d'écoulement s'étend. Deux pièces principales 116, 117 comprennent notamment les capteurs 111, 112 de pression débouchant dans les trous transversaux 141, 142 extrêmes de la filière 140. Une pièce intermédiaire 118 comprend le capteur 113 de pression débouchant dans le trou transversal 143 intermédiaire de la filière 140. Les pièces principales 116, 117, la pièce intermédiaire 118, ainsi que la pièce 120 de raccordement et la buse 130 d'injection sont assemblées, de façon démontable, par des vis 160.

La pièce intermédiaire 118 peut être omise. Dans ce cas, la filière utilisée ne comportera que deux trous transversaux extrêmes correspondant aux trous 141, 142 de la figure 1. On verra en effet dans la suite que la détermination de certains paramètres rhéologiques du matériau polymère mis en oeuvre n'exige la connaissance que de deux valeurs de la pression. La pièce 118 peut encore être remplacée par une autre pièce intermédiaire de longueur différente par rapport à l'axe du conduit d'écoulement.

Dans les deux cas, la filière 140 est alors remplacée par une filière de longueur différente, adaptée à la longueur de la nouvelle pièce intermédiaire. Cette nouvelle filière est d'une longueur correspondant à la nouvelle longueur du logement cylindrique 110a.

Il est également possible de démonter le corps 110 pour remplacer la filière 140 par une filière de diamètre intérieur différent.

La longueur des pièces 116, 117 et 118 selon l'axe du conduit d'écoulement est telle que l'ensemble constitué par le corps 110, la buse 130 et la pièce de raccordement est d'une longueur comparable à celle d'une buse classique. Le montage du rhéomètre sur la presse n'entraîne aucune perte de charge d'injection par rapport aux injections réalisées avec des buses classiques.

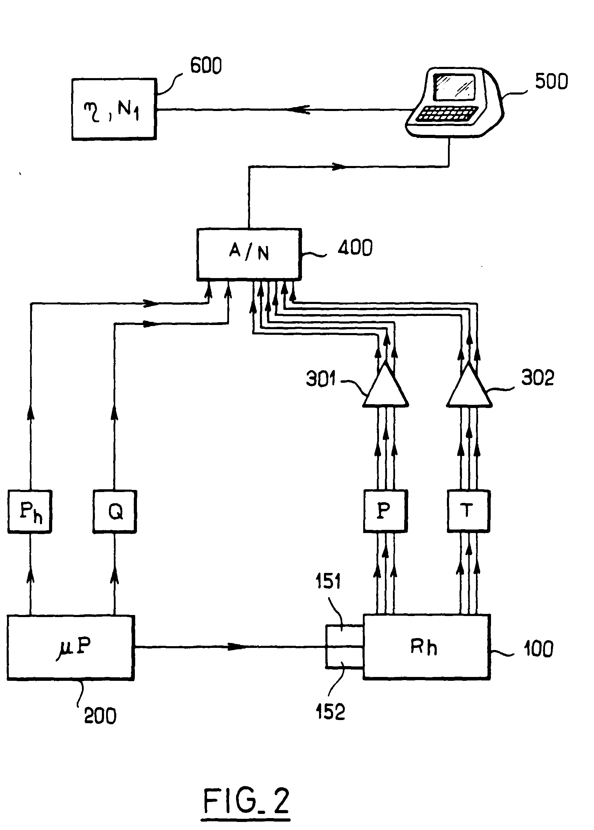

La figure 2 donne le schéma du traitement des données permettant d'accéder aux caractéristiques mécaniques du matériau polymère au cours de l'écoulement.

L'organe 200 de commande de la presse à injecter, un microprocesseur par exemple, impose à la vis d'alimentation une pression hydraulique $P_h$, un débit volumétrique Q et une température de fusion du matériau polymère considéré. D'autre part, comme cela a été dit plus haut, le microprocesseur 200 de commande contrôle le courant d'alimentation des manchons chauffants 151, 152 du rhéomètre.

Les mesures de pression et, éventuellement de température, fournies par le rhéomètre 100 sont amplifiées par les amplificateurs 301, 302 et appliquées, ainsi que le débit volumétrique Q et, au besoin, la pression hydraulique $P_h$, à un convertisseur analogique/numérique 400. Un microordinateur 500 traite les données ainsi numérisées selon un logiciel traduisant en langage informatique les développements mathématiques exposés ci-après. Les résultats obtenus, viscosité apparente et élasticité, sont transmises à un dispositif 600 de visualisation, imprimante ou table traçante.

On considère dans ce qui suit que l'écoulement dans la filière est un écoulement de cisaillement, établi et axisymétrique.

La contrainte de cisaillement $\tau_\omega$ à la paroi est donnée par la relation

$$\tau_\omega = \frac{R}{2} \cdot \frac{\Delta P}{L}$$

où R est le rayon de la filière et L sa longueur. $\Delta P$ est la perte de charge dans la filière. On observera que la mesure de $\Delta P$ ne nécessite que deux valeurs et donc deux capteurs de pression.

Dans le cas des fluides newtoniens, le taux de cisaillement à la paroi $\dot{\gamma}_\omega$ a pour expression :

$$\dot{\gamma}_\omega = \frac{4Q}{\pi R^3}$$

où Q est le débit volumétrique de la vis d'alimentation.

Dans le cas des fluides non newtoniens, il est nécessaire d'apporter une correction à la formule précédente pour obtenir un taux réel de cisaillement $\dot{\gamma}_{\omega r}$ défini par

$$\dot{\gamma}_{\omega r} = \frac{3}{4} \dot{\gamma}_\omega + \frac{1}{4} \dot{\gamma}_\omega \frac{d(\log \dot{\gamma}_\omega)}{d(\log \tau_\omega)}$$

Dans le cas des fluides dont le comportement rhéologique suit la loi puissance, l'indice de pseudoélasticité n peut s'écrire :

$$n = \frac{d(Ln \tau_\omega)}{d(Ln \dot{\gamma}_\omega)}$$

On obtient alors l'expression

$$\dot{\gamma}_{\omega r} = \frac{3n + 1}{4n} \dot{\gamma}_\omega$$

qui donne le taux réel de cisaillement à partir du taux apparent de cisaillement à la paroi.

La viscosité apparente corrigée $\eta$ s'écrit alors,

$$n = \frac{\tau_\omega}{\dot{\gamma}_{\omega r}}$$

L'élasticité $N_1$ des matériaux polymères à l'état fondu se caractérise en général par la première différence des contraintes normales :

$$N_1 = \tau_{11} - \tau_{22}$$

où $\tau_{11}$ désigne la contrainte normale dans la direction de l'écoulement et $\tau_{22}$ la contrainte normale dans la direction du cisaillement. L'élasticité peut s'exprimer en fonction de la pression de sortie Ps sous la forme :

$$N_1 = P_s \left(1 + \frac{d(LnPs)}{d(Ln\tau_\omega)}\right)$$

Dans le cas du rhéomètre de l'invention, la pression Ps en sortie de la filière 140 peut être déterminée par mesure directe ou par extrapolation linéaire des variations de la pression mesurée par les capteurs 111, 112, 113 en fonction de la position dans la filière. Le résultat sera d'autant plus précis que le nombre de capteurs de pression sera plus grand, ce qui explique l'intérêt de disposer de trois capteurs par exemple.

Le rhéomètre selon l'invention présente de nombreux avantages :

- il permet la détermination des propriétés rhéologiques différentes telles que la viscosité, l'élasticité et/ou la compressibilité, dans les conditions réelles de transformation par injection, sans perturbation du cycle de transformation et ce en un minimum de temps et avec une grande précision ;

- la présence de capteurs de pressions dans la filière (capillaire), à des positions bien précises et au contact de la matière polymère permet la mesure de la contrainte réelle de cisaillement à la paroi avec précision donc la viscosité de la matière est déterminée avec une grande précision ;

- la présence d'au moins trois capteurs dans la filière permet l'évaluation de l'élasticité des polymères, grandeur rhéologique à laquelle on n'a pas accès direct avec d'autres appareillages ;

- la possibilité de varier la longueur et le diamètre de la filière dans la présente invention ouvre un champs large d'études suivant la nature des polymères (produits de base, composites, mélanges, élastomères, etc.) donc permet l'accès à d'autres paramètres indispensables pour la transformation, tels que la compressibilité de la matière en fonction de la pression d'injection, le glissement à la paroi en particulier dans le cas des élastomères, les phénomènes de séparation de phases en fonction du taux de cisaillement dans le cas des mélanges de polymères, etc. ;

- la présence de capteurs de température au contact de la matière permet la mesure avec précision de la température du polymère, paramètre indispensable pour l'étude des caractéristiques rhéologiques de la matière et qui conditionne en grande partie la transformation ; ainsi la présence de deux capteurs de température en amont et en aval de la filière (170 et 171) permet l'étude du phénomène d'autoéchauffement de la matière ;

- le système d'acquisition de données (figure 2) permet non seulement l'enregistrement et la présentation des propriétés rhéologiques mais, également la détermination d'une loi de comportement du matériau basée sur les données expérimentales ; cette loi est indispensable pour la modélisation de l'écoulement des polymères au cours de la transformation ;

- ce système permet, en présence des capteurs dans le moule, d'étudier le comportement de la matière dans les canaux d'alimentation et dans l'empreinte du moule.

## Revendications

1. Rhéomètre (100) destiné à être disposé en sortie d'une vis d'alimentation d'une machine de transformation d'un matériau polymère, comprenant un corps (110) de rhéomètre dont une première extrémité est munie d'une pièce (120) de raccordement mécanique à ladite vis d'alimentation et dont une deuxième extrémité, opposée à la première, est munie d'une buse (130) d'injection du matériau polymère, ledit corps (110) de rhéomètre présentant un conduit pour l'écoulement dudit matériau polymère et au moins deux capteurs (111, 112, 113) de pression placés le long dudit conduit, ledit conduit étant au moins partiellement constitué par une filière (140) présentant un trou longitudinal (144) cylindrique d'écoulement, caractérisé en ce que la filière (140) présente au moins deux trous transversaux (141, 142, 143) débouchant dans ledit trou d'écoulement et destinés à recevoir respectivement lesdits capteurs (111, 112, 113) de pression au débouché du trou (144) d'écoulement, et en ce que le corps (110) du rhéomètre comprend deux pièces principales (116, 117) et une pièce intermédiaire (118) assemblées de façon démontable et recevant la filière, de sorte que la pièce intermédiaire peut être omise ou remplacée par une pièce intermédiaire de hauteur différente, la filière pouvant être remplacée par une filière correspondante de hauteur et/ou de diamètre différent(s).

2. Rhéomètre selon la revendication 1, caractérisé en ce que ledit corps (110) de rhéomètre comporte en outre au moins un trou (114, 115) de logement destiné à recevoir un capteur de température.

3. Rhéomètre selon l'une des revendications précédentes, caractérisé en ce que le diamètre du trou d'écoulement (144) de la filière est inférieur ou égal à 2,5 mm.

4. Rhéomètre selon la revendication 3, caractérisé en ce que le diamètre du trou d'écoulement (144) de la filière est compris entre 2 et 2,5 mm.

5. Rhéomètre selon la revendication 2, caractérisé en ce que ladite pièce (120) de raccordement comporte un trou (121) de logement destiné à recevoir un capteur de température.

6. Rhéomètre selon l'une des revendications 2 et 5, caractérisé en ce que le corps (110) du rhéomètre comporte au moins deux trous (171, 172) de logement destiné à recevoir un capteur de température et débouchant dans le conduit respectivement en amont et en aval de la filière (140).

7. Rhéomètre selon l'une des revendications précédentes, caractérisé en ce que la filière (140) présente au moins trois trous transversaux (141, 142, 143) débouchant dans le trou d'écoulement et recevant des capteurs de pression (111, 112, 113) au débouché dudit trou (144) d'écoulement.

8. Rhéomètre selon la revendication 7, caractérisé en ce que les capteurs de pression (111, 112, 113) sont reliés à une unité de traitement qui détermine en fonction des mesures de pression fournies par ces capteurs la viscosité apparente et l'élasticité du matériau polymère.

**Patentansprüche**

1. Rheometer (100) zur Anordnung am Ausgang einer Zuführeinrichtung bzw. - schnecke einer Maschine zur Verarbeitung eines Polymer-Materials, mit einem Rheometerkörper (110), bei dem ein erstes Ende mit einem Teil (120) zur mechanischen Verbindung mit der Zuführeinrichtung versehen ist und bei dem ein dem ersten gegenüberliegendes zweites Ende mit einer Düse (130) zum Einspritzen des Polymer-Materials versehen ist, wobei der Rheometerkörper (110) einen Kanal für den Abfluß des Polymer-Materials und mindestens zwei entlang des Kanals angeordnete Drucksensoren (111, 112, 113) hat und der Kanal zumindest teilweise durch eine Düse (140) gebildet wird, die ein zylindrisches Abflußloch (144) in Längsrichtung hat, dadurch gekennzeichnet, daß die Düse (140) mindestens zwei Löcher (141, 142, 143) in Querrichtung hat, welche in das Abflußloch münden und zum Aufnehmen der jeweiligen Drucksensoren (111, 112, 113) an der Mündung des Abflußlochs (144) bestimmt sind, und daß der Reometerkörper (110) zwei Hauptstücke (116, 117) und ein Zwischenstück (118) aufweist, die zerlegbar zusammengebaut sind und die Düse enthalten, so daß das Zwischenstück weggelassen oder durch ein Zwischenstück unterschiedlicher Höhe ersetzt werden kann, wobei die Düse durch eine entsprechende Düse mit unterschiedlicher Höhe und/oder unterschiedlichem Durchmesser ersetzt werden kann.

2. Rheometer nach Anspruch 1, dadurch gekennzeichnet, daß der Rheometerkörper (110) darüberhinaus mindestens ein Aufnahmeloch (114, 115) zur Aufnahme eines Temperatursensors aufweist.

3. Rheometer nach einem der vorhergehenden Ansprüche, dadurch gekennzeichnet, daß der Durchmesser des Abflußlochs (144) der Düse kleiner oder gleich 2,5 mm ist.

4. Rheometer nach Anspruch 3, dadurch gekennzeichnet, daß der Durchmesser des Abflußlochs (144) der Düse zwischen 2 und 2,5 mm ist.

5. Rheometer nach Anspruch 2, dadurch gekennzeichnet, daß das Verbindungsteil (120) ein Aufnahmeloch (121) zur Aufnahme eines Temperatursensors aufweist.

6. Rheometer nach einem der Ansprüche 2 und 5, dadurch gekennzeichnet, daß der Rheometerkörper (110) mindestens zwei Aufnahmelöcher (171, 172) aufweist, die zur Aufnahme eines Temperatursensors dienen und in den Kanal stromaufwärts bzw. stromabwärts der Düse (140) münden.

7. Rheometer nach einem der vorhergehenden Ansprüche, dadurch gekennzeichnet, daß die Düse (140) mindestens drei Löcher (141, 142, 143) in Querrichtung hat, welche in das Abflußloch münden und Drucksensoren (111, 112, 113) an der Mündung des Abflußlochs (144) aufnehmen.

8. Rheometer nach Anspruch 7, dadurch gekennzeichnet, daß die Drucksensoren (111, 112, 113) mit einer Verarbeitungseinheit verbunden sind, welche in Abhängigkeit von den durch diese Sensoren gelieferten Druckmeßwerten die scheinbare Viskosität und Elastizität des Polymer-Materials bestimmt.

**Claims**

1. A rheometer (100) for placing at the outlet of a feed screw of a machine for transforming polymer material, the rheometer comprising a body (110) having a first end provided with a piece (120) for mechanical coupling to said feed screw and having a second end, remote from the first, provided with a nozzle (130) for injecting the polymer material, said rheometer body (110) having a duct for conveying a flow of said polymer material and at least two pressure sensors (111, 112, 113) placed along said duct, said duct being constituted at least in part by a die (140) presenting a cylindrical longitudinal flow hole (144), the rheometer being characterized in that the die (140) presents at least two transverse holes (141, 142. 143) opening out into said flow hole and designed respectively to receive said pressure sensors (111, 112, 113) at their outlets into the flow hole (144), and in that the rheometer body (110) comprises two main parts (116, 117) and an intermediate part (118) assembled together in releasable manner and receiving the die, such that the intermediate part can be omitted or replaced by an intermediate part of different height, the die being replaceable by a corresponding die of different height and/or diameter.

2. A rheometer according to claim 1, characterized in that said rheometer body (110) further includes at least one housing hole (114, 115) for receiving a temperature sensor.

3. A rheometer according to either preceding claim, characterized in that the diameter of the flow hole (144) of the die is less than or equal to 2.5 mm.

4. A rheometer according to claim 3, characterized in that the diameter of the flow hole (144) of the die lies in the range 2 mm to 2.5 mm.

5. A rheometer according to claim 2, characterized in that said coupling piece (120) includes a housing hole (121) for receiving a temperature sensor.

6. A rheometer according to claim 2 or 5, characterized in that the rheometer body (110) includes at least two housing holes (171, 172) for receiving temperature sensors and opening out into the duct respectively upstream and downstream from the die (140).

7. A rheometer according to any preceding claim, characterized in that the die (140) presents at least three transverse holes (141, 142, 143) opening out into the flow hole and receiving pressure sensors (111, 112, 113) at their outlets into said flow hole (144).

8. A rheometer according to claim 7, characterized in that the pressure sensors (111, 112, 113) are connected to a processor unit which determines the apparent viscosity and the elasticity of the polymer material as a function of the pressure measurements provided by said sensors.

## FIG_1

FIG_2